# EUROPEAN PATENT APPLICATION

(11) **EP 0 564 176 A1**
(43) Date of publication of application: **06.10.1993**
(21) Application number: 93302337.6
(22) Date of filing: 26.03.1993
(51) Int. Cl.: A61B 17/14

(54) **Ankle clamp**

(30) Priority: 30.03.1992 US 859666
(71) Applicant: SMITH & NEPHEW RICHARDS, INC., Memphis, Tennessee 38116 (US)
(72) Inventor: Steele, John, Mephis, Tennessee 38120 (US)
(74) Representative: Gilholm, Stephen Philip

(57) **Abstract**

An ankle clamp (10) apparatus for use with tibial cutting instruments has a frame (11) with pivoting arms (13,14) attached for gripping a patient's ankle during use. The arms (13,14) can be held open with latches (35,36) prior to placement and quickly released to grip the patient's leg by depressing the latches (35,36). The device can be operated by a surgeon using only one hand to depress the latches (35,36).

## Description

The present invention relates to orthopaedic instrumentation and more particularly relates to an ankle clamp apparatus which allows a surgeon to use one hand to attach the ankle clamp as part of an overall tibial alignment assembly. More particularly, the present invention relates to an improved ankle clamp apparatus with a locking feature that secures movable arm portions of the clamp in an open position until the clamp is positioned for use at which time the locking feature may be disengaged from the arms, allowing the clamp to tighten around the ankle of the patient.

During total knee replacement, the upper or proximal portion of the tibia is cut away using a saw such as a reciprocal saw. The surgeon places the saw on the top of a cutting block that is positioned at the proximal end of the tibia. The cutting block provides a surface that supports the saw blade in a desired, steady position. Placement of tibia instrumentation and cutting block is critical because the block must be positioned correctly and held firm during the entire cutting operation so that an accurate cut is made.

One way to mount the cutting block and its associated instrumentation is to actually mount the cutting block to an intermedullary canal, that occupies the hollowed inside portion of the tibia, so that the cutting block lies adjacent to the bone to be sawed.

A second way to correctly align the cutting block is through an external or extramedullary alignment assembly. These devices include extendable telescoping, or solid rod assemblies with a lower end portion carrying an ankle clamp that grasps the patient's ankle. By holding the patient's leg externally and very firmly with assist from the ankle clamp, the cutting block can be positioned and held steady during the cutting so that the cuts are made precisely by the surgeon.

Extramedullary alignment assemblies include the extendable telescoping rod for the purpose of adjusting the entire instrumentation assembly to the length of a particular patient's leg. Similarly, extramedullary alignment assemblies which include a solid non-extendable rod permit the cutting block to slide thereon and be selectively positioned to a desired clamping location. Additionally, lateral adjustments are available for moving the extramedullary alignment assembly away from or toward the patient's tibia.

Presently, ankle clamps are commercially available which include a frame (also known as a "Y-block") with a pair of arms pivotally attached to the frame on opposite sides. These arms are spring loaded so that they are biased to a closed clamping position until opened by the surgeon. These prior art clamps do not provide a means to maintain the arms in an open position, and because there are two arms, two hands are required to open these prior art type ankle clamps.

The object of the present invention is to provide an improved ankle clamp that allows a surgeon to use one hand to attach the ankle clamp for a tibial alignment assembly. The object of the present invention is achieved by using a mechanism that locks the arms of the clamp in an open non ankle-engaging position until the clamp is positoned by the surgeon at the patient's ankle. The surgeon can then relock the arms simultaneously by depressing release latches so that the clamp tightens quickly around the ankle to a closed ankle engaging position.

The present invention provides an ankle clamp apparatus for supporting tibial cutting instruments comprising a frame having a first fixed arm and a second fixed arm; and a first and second movable arm each movable arm having a free end and an attachment end, the first and second movable arms being mounted at the attachment end to the first and second fixed arm respectively and movable between an open non-ankle engaging position and a closed ankle engaging position, characterised in that the apparatus comprises latch means for maintaining the movable arm in the open position, the latch means being provided with a release means which disengages a selected moveable arm from the open non-ankle engaging position.

The ankle clamp frame, is preferably substantially Y-shaped having a first and second fixed arm each arm having a free end portion. The frame is adapted so that there is a gap between the fixed arms that accepts the patient's ankle during use.

Suitably a pivot is provided at the free end portion of the fixed arms. Each pivot forms an attachment position for each movable arm. A movable arm may be attached to each of the spaced apart sides or fixed arms of the Y-block frame at the pivots. The movable arms are movable between an open non-ankle engaging position and a closed ankle-engaging position. Each movable arm may be biased with spring means to assume the closed position which grips the patient's ankle during use. The spring means may be any suitable spring known to those skilled in the art. A preferred spring means is a helical or coiled spring.

Each of the movable arms includes a free end portion spaced from it's pivot, and a notched end portion adjacent the fixed arm pivot. The notched end suitably comprises a plurality of notches. During operation, each movable arm is rotated toward an open position, tightening its helical spring. The spring may be pivotally attached to the fixed arm. The free end of the spring is attached to the latch means. Once the arm is rotated beyond a full open position, a notched end portion on the movable arm is exposed into which the latch means seats. The notches cooperate with the latch means to secure the movable arms in the open position. Aptly the latch means may comprise a single latch member. More suitably the latch means comprises more than one latch member. Preferably the latch means comprises two latch members, one of the latch members being mounted on one of the fixed arms and the other being mounted on the other fixed arm. Each of the latches may have an externally positioned pressure plate surface that can be manually depressed for releasing the latch from the respective movable arm. The latch members are movable between latching and unlatching positions.

With the ankle clamp in the open position, the surgeon positions the clamp on the ankle. Next, the surgeon manually depresses or squeezes the latches which move clear of the notch thus allowing the movable arms to move to the closed position. The ankle clamp apparatus as described allows the surgeon to use one hand to attach the ankle clamp for a tibial alignment assembly, simplifying the overall tibial cutting surgical procedure.

For a further understanding of the nature and objects of the present invention, reference should be had to the following detailed description taken in conjunction with the accompanying drawings, in which like parts are given like reference numerals, and wherein:
FIGURE 1 is a plan view of the preferred embodiment of the ankle clamp apparatus of the present invention;
FIGURE 2 is a fragmentary plan view of the preferred embodiment of the ankle clamp apparatus of the present invention illustrating the movable arms in the closed ankle-engaging position;
FIGURE 3 is a fragmentary plan view of the preferred embodiment of the apparatus of the present invention illustrating the movable arms in the open non-ankle engaging position;
FIGURE 4 is a front schematic elevational view of the preferred embodiment of the apparatus of the present invention illustrating its attachment to a patient's leg and ankle;
FIGURE 5 is a side elevational schematic view of the preferred embodiment of the apparatus of the present invention illustrating its position during use with regard to the patient's leg and ankle;
FIGURE 6 is a fragmentary elevational view illustrating the arms in closed position about a patient's ankle.

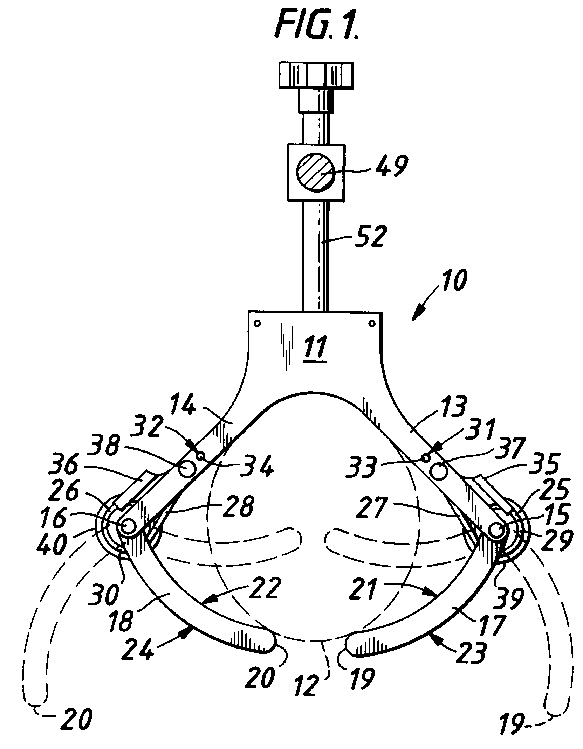

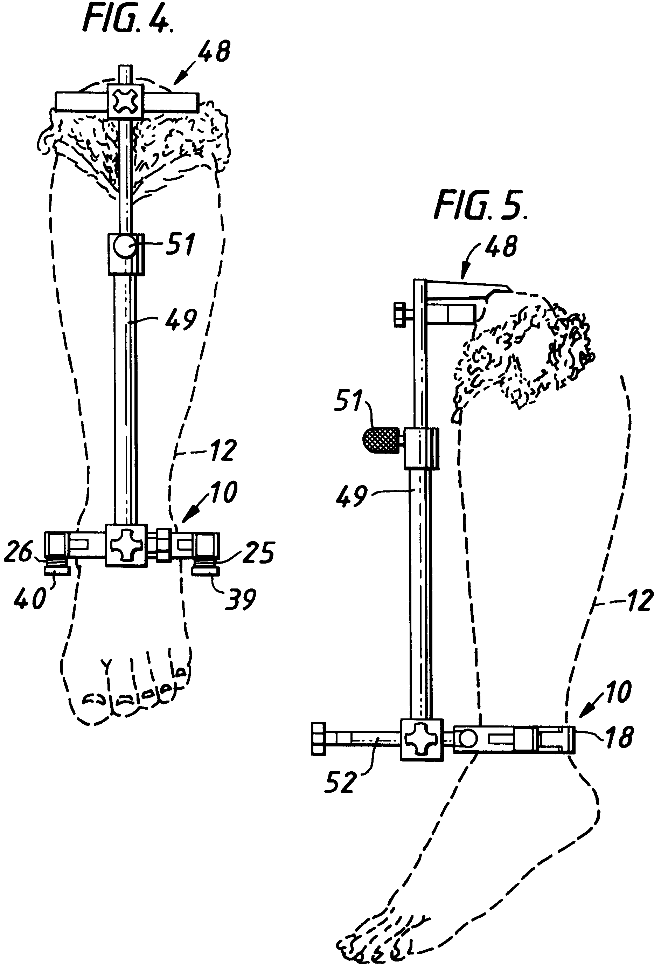

Figures 1-6 illustrate the preferred embodiment of the apparatus of the present invention designated generally by the numeral 10. Ankle clamp apparatus 10 includes a frame 11 preferably in the shape of a Y-block as shown in Figure 1. The frame 11 provides an open recess between a pair of fixed arms 13, 14. The recess accommodates a patient's leg 12 at the ankle area as shown in Figures 1, 4 and 5 when the ankle clamp 10 is in the closed ankle-engaging position as shown in hard lines in Figures 1 and 5. Fixed arms 13, 14 are angularly oriented with respect to each other, by a measure of approximately ninety degrees (90°). Each fixed arm 13, 14 provides a pivot 15, 16 respectively that pivotally supports movable arms 17, 18 respectively.

Each movable arm 17, 18 provides respective free end portions 19, 20, and is preferably curved having outer concave surfaces 21, 22 and inner convex surfaces 23, 24 respectively. A pair of coiled or helical springs 25, 26 bias each of the movable arms 17, 18 into the closed ankle-engaging position as shown in hard lines in Figure 1.

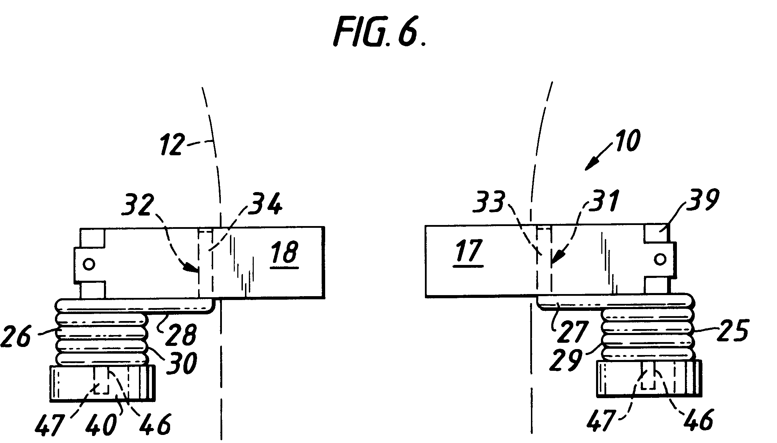

Each coiled spring 25, 26 comprisese a straight portion 27, 28, a coiled portion 29, 30, and a spring anchor 47 which fits into a spring peg opening 46 in a spring peg 39, 40 (see Figure 6). The straight portions 27, 28 of springs 25, 26 having spring attachments 33, 34 respectively that register in spring attachment openings 31, 32. Openings 31, 32 are cylindrical openings having an internal diameter slightly larger than the external diameter of spring attachments 33, 34.

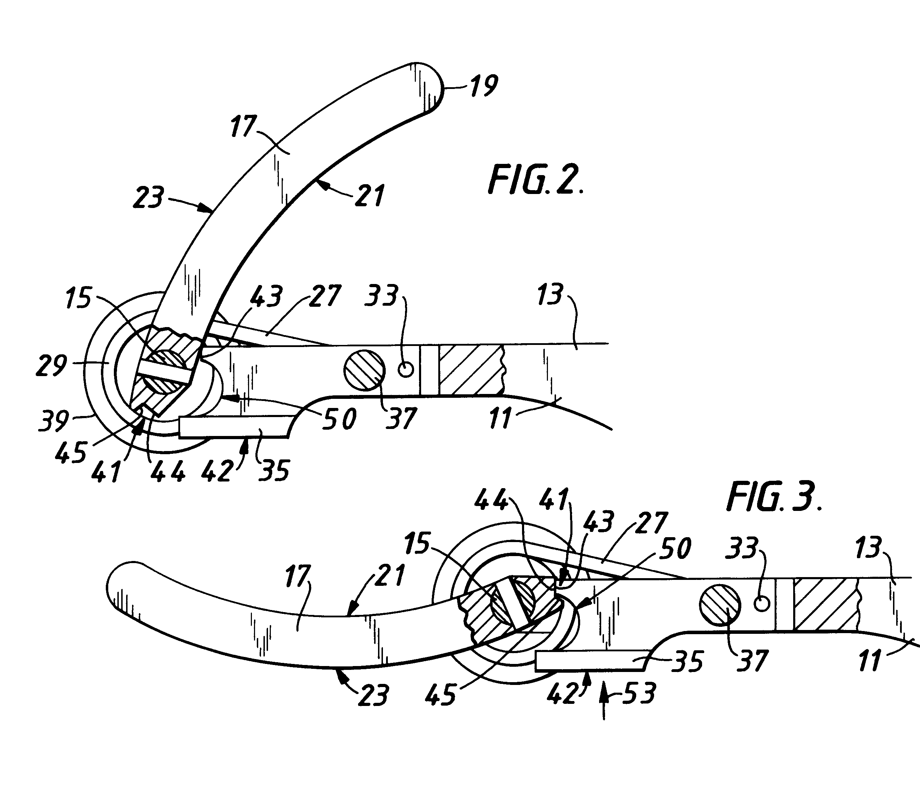

A pair of latches 35, 36 are provided respectively upon the fixed arms 13, 14. Each latch 35, 36 secures its respective movable arm 17, 18 in the open position (see Figure 3). The coiled springs 25, 26 fit around spring pegs 39, 40 respectively which are mounted at the free ends of fixed arms 13, 14 as shown in Figure 1. Each latch 35, 36 has an opening substantially the same diameter as its associated spring attachment. The spring attachments 33, 34 pass through the opening in each respective latch 35, 36 and through the larger openings 31, 32. This allows a small amount of travel of each spring attachment 33, 34 in the openings 31, 32. Because the openings in each latch 35, 36 are substantially the same size as the spring attachments 33, 34, the latches 35, 36 are urged by the springs 25, 26 to move to the latching or open non-ankle engaging position, as shown in Figure 3. Each spring attachment 33, 34 moves in its opening 31, 32 toward the recess between fixed arms 13, 14 causing each latch 35, 36 to pivot about its pivot 37, 38 and rotating free end 43 away from the recess area between fixed arms 13, 14 (ie. latching or open non-ankle engaging position).

Figures 2 and 3 show a single fixed arm 13 and its associated movable arm 17 for purposes of illustrating the operation of a latch 35 with regard to the movement of movable arm 17 between closed (Figure 2) and open (Figure 3) positions.

In Figure 2, the latch 35 provides a latch free end 43 that communicates with latch pressure plate 42, both spaced away from the pivot 37. The pressure plate 42 is depressed or squeezed by the surgeon in order to release the movable arm 17 so that it moves from the open position of Figure 3 to the closed position of Figure 2. The position of movable arm 17 in Figure 2 is achieved if the device is moved to the closed position when a patient's ankle is not being gripped. Otherwise, the patient's leg 12 forms a stop for receiving the moving arms 17, 18 as shown in Figure 1. In Figure 1, the full open position as well as the full closed position of the arms is shown in phantom lines with the actual operative gripping position about a patient's leg 12 being shown in hard lines for arm 17.

Movable Arm 17 provides a recess or notch 41 receptive of latch free end 43 as shown in Figure 3. The notch 41 is comprised of a pair of angularly intersecting flat notch surfaces 44, 45 which are angled approximately ninety degrees (90°) with respect to each other. During use, the surgeon pulls arms 17 and 18 from the closed position of Figure 2 into the open position of Figure 3. In so doing, the latch free end 43 registers with the notch 41 and abuts the surface 44 as shown in Figure 3. At this time, the surgeon releases the movable arm 17 or 18 and coiled spring 25 or 26 holds the surface 44 tightly against the surface 43 in attempting to close the movable arm 17. However, the latch 35 free end 43 engages the surface 44 and prevents the movable arm 17 from moving toward the closed position of Figure 2.

In order to move the arms 17, 18 into the closed position of Figure 2, the surgeon presses against the pressure plate 42 rotating the latches 35, 36 in the direction of arrow 53 in Figure 3, thus rotating the latch 35 about its pivot 37. This movement of the latch 35 about its pivot 37 disengages the surface 43 from the surface 44 and places the notch 41 adjacent the concave surface 50 portion of latch 35 which allows the movable arm 17 or 18 to freely pivot toward the closed position of Figure 2. The arm 17 concave surface 21 eventually engages the latch free end 43 as shown in Figure 2 which defines the innermost possible position of the pivoting arms 17, 18.

In Figures 1, 2 and 3, the ankle clamp apparatus 10 is shown as part of an overall tibial instrumentation assembly that includes tibial cutting block assembly 48 and telescoping rod assembly 49.

Because many varying and different embodiments may be made within the scope of the inventive concept herein taught, and because many modifications may be made in the embodiments herein detailed in accordance with the descriptive requirement of the law, it is to be understood that the details herein are to be interpreted as illustrative and not in a limiting sense.

## Claims

1. An ankle clamp apparatus for supporting tibial cutting instruments comprising a frame having a first fixed arm and a second fixed arm; and a first and second movable arm each movable arm having a free end and an attachment end, the first and second movable arms being mounted at the attachment end to the first and second fixed arm respectively and movable between an open non-ankle engaging position and a closed ankle engaging position, characterised in that the apparatus comprises latch means for maintaining the movable arms in the open position, the latch means being provided with a release means which disengages a selected moveable arm from the open non-ankle engaging position.

2. An ankle clamp apparatus as claimed in claim 1 wherein the frame is substantially Y-shaped, the first and second fixed arm each having a free end portion; an outer non-ankle engaging side portion and an inner ankle engaging side portion

3. An ankle clamp apparatus as claimed in claim 2 wherein the movable arms are pivotally mounted at the free end portion of the fixed arms.

4. An ankle clamp apparatus as claimed in any of the preceding claims wherein the latch means is carried by the frame.

5. An ankle clamp apparatus as claimed in any of the preceding claims further comprising means for biasing the movable arms to the closed position.

6. An ankle clamp apparatus as claimed in claim 5 wherein the biasing means includes spring means carried by the frame for urging the movable arms to move to the closed position.

7. An ankle clamp apparatus as claimed in any of the preceding claims wherein each movable arm has a end portion comprising a plurality of notches at the end opposite to its free end.

8. An ankle clamp apparatus as claimed in claim 7 wherein the latch means engages the notched end portion when the movable arms are in the open position.

9. An ankle clamp apparatus as claimed in claim 6 wherein the biasing means includes a spring carried on each of the fixed arms.

10. An ankle clamp apparatus as claimed in any of the preceding claims wherein the fixed arms are integral and are angularly oriented, and an open recess is provided between the fixed arms that is receptive of the patient's ankle during use.

11. An ankle clamp apparatus for supporting tibial cutting instruments, comprising:
a) an ankle clamp frame having left and right side portions with a gap therebetween;
b) a pair of arms, each attached to one of the pair of side portions between open and closed positions, and each arm biased to assume the closed position;
c) latch means for holding the arms in the open position;
d) release means for moving a selected arm to the closed position.

12. An ankle clamp apparatus as claimed in claim 11 wherein the frame includes a yoke that is generally Y-shaped.

13. An ankle clamp apparatus as claimed in any of the preceding claims wherein the latch means engages a portion of the fixed arm adjacent the attachment end of the movable arm.

14. An ankle clamp apparatus as claimed in any one of the preceding claims wherein each movable arm has a notched end portion opposite its free end portion.

15. An ankle clamp apparatus as claimed in claim 14 wherein the latch means engages the notched end portion when the movable arms are in the open ankle non-engaging position.

16. An ankle clamp apparatus as claimed in any of the preceding claims wherein there is further included a spring for moving the movable arms to the closed position.

17. An ankle clamp apparatus as claimed in claim 16 wherein the spring includes coil springs for urging the movable arms to move to the closed position.

18. An apparatus of claim 17 wherein a coil spring is carried respectively on each of the outer side portions of the frame.

19. An ankle clamp apparatus as claimed in any of the preceding claims wherein the frame includes a yoke with a pair of integrally connected fixed arms that are angularly oriented, and an open recess is provided between the fixed arms that is receptive of the patient's ankle during use.

20. An ankle clamp apparatus as claimed in any of the preceding claims wherein each fixed arm is an elongated curved member.

21. An ankle clamp apparatus as claimed in any of the preceding claims wherein the latch means comprises a pair of latch members mounted respectively on the outer side portion of the frame.

22. An ankle clamp apparatus as claimed in claim 7 wherein the latch means comprises a latch member mounted respectively on each of the outer side portions of the frame, the latch members engaging the notches when the movable arms are in the open ankle non-engaging position.

23. An ankle clamp apparatus as claimed in claim 21 wherein the latch members are pivotally mounted to the frame.

24. An ankle clamp apparatus as claimed in any of claims 2 to 23 wherein there is further provided a spring peg mounted at the free end of each fixed arm, each spring peg supporting a coil spring.

25. An ankle clamp apparatus as claimed in claim 24 wherein each coil spring is anchored at one end portion to the spring peg and at its other end portion to a fixed arm.

26. The apparatus of claim 25 wherein the latches are biased to assume the latching position.

27. The apparatus of claim 26 wherein the springs bias each latch to the latching position.

28. An ankle clamp for supporting tibial cutting instruments, comprising:
a) an ankle clamp frame having a pair of angularly intersecting portions with a gap therebetween that accepts a patient's ankle during use;
b) a pair of pivots mounted on the frame and respectively at the pair of intersecting portions;
c) a pair of movable arms, each with a free end portion and an attachment end portion, and the arms are pivotally attached respectively to the pair of intersecting portions at a pivot, and movable between open and closed positions, the frame and arms defining an assembly during use;
d) latch means carried by the assembly of the frame and the attached arms for holding the arms in the open position;
e) the latch means including quick release means for moving a selected arm to the closed position.
